# EUROPEAN PATENT APPLICATION

(11) **EP 4 778 528 A1**
(43) Date of publication of application: **22.07.2026**
(21) Application number: 23951888.9
(22) Date of filing: 15.09.2023
(51) Int. Cl.: A61K 31/704, A61K 36/258, A61P 43/00, A61P 25/28

(54) **USE OF GINSENG EXTRACT IN PREPARATION OF COMPOSITION FOR RESISTING FATIGUE OR IMPROVING COGNITIVE FUNCTION PERFORMANCE**

(71) Applicant: TCI Co., Ltd, Taipei 11494 (CN)
(72) Inventor: LIN, Yung-Hsiang, 11494 Taipei Taiwan (CN); LIN, Hsiao-Nai, 11494 Taipei Taiwan (CN)
(74) Representative: Viering, Jentschura & Partner mbB Patent- und Rechtsanwälte
(86) International application number: PCT/CN2023/119064
(87) International publication number: WO 2025/054956

(57) **Abstract**

A use of a ginseng extract for preparing a composition for anti-fatigue or improving cognitive function is provided. The ginseng extract is obtained by water extraction from roots of *Panax ginseng* C. A. Meyer and contains a relatively high content of ginsenoside Rg3. The composition is capable of enhancing mitochondrial activity, promoting skeletal muscle cell proliferation, improving hypoxia tolerance, increasing blood oxygen saturation, reducing oxidative stress, and enhancing cognitive performance, including judgment ability and short-term memory, in a subject in need thereof.

## Description

### BACKGROUND

### Technical Field

The present invention relates to use of a ginseng extract, and in particular, to use of a ginseng extract prepared from a ginseng for anti-fatigue or improving cognitive function.

### Related Art

Fresh ginseng newly harvested from soil contains a high moisture content and is therefore difficult to preserve and is rarely available on the market. Such fresh ginseng is only occasionally used as a food ingredient in areas close to its place of origin. In general, ginseng is subjected to different processing and preparation procedures, whereby fresh ginseng is processed into red ginseng, white ginseng, or taegeuk ginseng.

Although it is generally believed that red ginseng, white ginseng, and taegeuk ginseng exhibit superior effects and higher ginsenoside contents than fresh ginseng, each of red ginseng, white ginseng, and taegeuk ginseng possesses distinct characteristics and applicability. Differences in processing and preparation methods further result in variations in the quality of red ginseng, white ginseng, and taegeuk ginseng.

### SUMMARY

In view of the foregoing, in order to avoid over-processing or unknown effects caused by processing procedures, the present invention provides a ginseng extract obtained from ginseng without undergoing drying or heat-processing steps, thereby substantially preserving the original bioactive components of ginseng. The ginseng extract can be applied for preparing an anti-fatigue composition or a composition for improving cognitive function.

In some embodiments, a use of a ginseng extract for preparing an anti-fatigue composition is provided, wherein the ginseng extract is extracted from *Panax ginseng* C. A. Meyer using water as an extraction solvent.

In some embodiments, the ginseng extract enhances mitochondrial activity.

In some embodiments, the ginseng extract promotes proliferation of skeletal muscle cells.

In some embodiments, a use of a ginseng extract for preparing a composition for improving cognitive function is provided, wherein the ginseng extract is extracted from *Panax ginseng* C. A. Meyer using water as an extraction solvent.

In some embodiments, the ginseng extract improves hypoxia tolerance.

In some embodiments, the ginseng extract improves blood oxygen saturation level.

In some embodiments, the ginseng extract improves lipid peroxidation by increasing a level of malondialdehyde (MDA).

In some embodiments, the ginseng extract improves total antioxidant capacity (TAC).

In some embodiments, the composition is a food composition, and the composition comprises at least 5 mL of the ginseng extract. That is, an effective daily amount of the ginseng extract is 5 mL per day.

In some embodiments, the ginseng extract comprises at least 13.3 mg of ginsenoside Rg3 per 100 mL.

In summary, the ginseng extract of any embodiment can be used for preparing an anti-fatigue composition or a composition for improving cognitive function. The ginseng extract of any embodiment can further be used for enhancing mitochondrial activity, promoting proliferation of skeletal muscle cells, improving hypoxia tolerance, improving blood oxygen saturation level, improving lipid peroxidation by increasing a level of malondialdehyde (MDA), and improving total antioxidant capacity (TAC). In any embodiment, the ginseng extract comprises at least 13.3 mg of ginsenoside Rg3 per 100 mL.

### BRIEF DESCRIPTION OF THE DRAWINGS

FIG. 1 is a fingerprint chromatogram of a ginseng extract;
FIG. 2 is a bar chart showing relative content of ginsenoside Rg3;
FIG. 3 is a bar chart showing relative content of total alkaloid;
FIG. 4 is a bar chart showing relative antioxidant efficiency;
FIG. 5 is a bar chart showing relative proliferation rate of skeletal muscle cells ;
FIG. 6 is a bar chart showing relative proliferation rate of skeletal muscle cell experiment ;
FIG. 7 is a bar chart showing blood oxygen saturation level test results obtained from a human trial of the ginseng extract. ;
FIG. 8 is a bar chart showing lipid peroxidation indicator test results obtained from a human trial of the ginseng extract.;
FIG. 9 is a bar chart showing total antioxidant capacity (TAC) test results obtained from a human trial of the ginseng extract.;
FIG. 10 is a bar chart showing total time required to complete the test results obtained from a human trial of the ginseng extract;
FIG. 11 is a bar chart showing the number of correct answers obtained from a human trial of the ginseng extract.; and
FIG. 12 is a bar chart showing answer accuracy test results obtained from a human trial of the ginseng extract.

### DETAILED DESCRIPTION

As used herein, the term 'extract' refers to a product obtained through extraction. This extract can be supplied as a solution dissolved in a solvent or as a concentrate or essence that is free of, or substantially free of, the solvent.

In this document, the term 'ginseng' refers to the fleshy root of fresh ginseng (Panax ginseng), also known as Korean ginseng. In certain instances, it refers to a ginseng root that has not been dried or steamed. In some embodiments, the term refers to a root that has been frozen or refrigerated within seven days of harvesting. The root may be used whole or chopped, diced, crushed, ground, milled or otherwise processed to modify its size and physical integrity.

In some embodiments, the ginseng is ginseng produced in northeastern China. In some embodiments, the ginseng is artificially cultivated ginseng having a cultivation period of less than five years.

In some embodiments, the ginseng extract is obtained by extracting ginseng using water as an extraction solvent. In some embodiments, the ginseng extract is obtained by performing a heated aqueous extraction of ginseng. In some embodiments, the heated extraction comprises heating a mixture of water and ginseng at a temperature of about 85±5° C for about 50 to 80 minutes. In other words, the extraction temperature is about 85±5° C, and the extraction time is about 50 to 80 minutes. In some embodiments, the extraction temperature is about 85° C, and the extraction time is about 60 minutes. In some embodiments, the extraction is performed by immersing the ginseng in heated water.

In some embodiments, a weight ratio of water to ginseng ranges from about 1:5 to about 1:15. In some embodiments, the weight ratio of water to ginseng is about 1:5.

In some embodiments, the ginseng extract is obtained by extracting ginseng using water as the solvent, producing a primary extract. This is then filtered through a mesh screen to remove solid residues. For example, this screen could be a 400-mesh screen.

In some embodiments, the ginseng extract comprises at least 13.3 mg of ginsenoside Rg3 per 100 mL.

In some embodiments, the ginseng extract is used for preparing an anti-fatigue composition. In some embodiments, the ginseng extract achieves an anti-fatigue effect by enhancing mitochondrial activity. In some embodiments, the ginseng extract achieves an anti-fatigue effect by promoting proliferation of skeletal muscle cells. In other words, the ginseng extract may promote conversion of muscular strength.

In some embodiments, the ginseng extract is used for preparing a composition for improving cognitive function. In some embodiments, the ginseng extract achieves improvement of cognitive function by improving hypoxia tolerance. In some embodiments, the ginseng extract achieves improvement of cognitive function by improving blood oxygen saturation level. In some embodiments, the ginseng extract achieves improvement of cognitive function by improving lipid peroxidation by increasing a level of malondialdehyde (MDA). In some embodiments, the ginseng extract achieves improvement of cognitive function by improving total antioxidant capacity (TAC).

In some embodiments, the composition is a food composition, and the composition comprises at least 5 mL of the ginseng extract.

In some embodiments, the foregoing composition may be a pharmaceutical composition. In other words, the pharmaceutical composition comprises an effective amount of the ginseng extract.

In some embodiments, the foregoing pharmaceutical composition may be formulated into a dosage form suitable for enteral or oral administration using techniques well known to those skilled in the art. Such dosage forms include, but are not limited to, tablets, troches, lozenges, pills, capsules, dispersible powders or granules, solutions, suspensions, emulsions, syrups, elixirs, slurries, or the like.

In some embodiments, the foregoing pharmaceutical composition may be formulated into a dosage form suitable for parenteral or topical administration using techniques well known to a person skilled in the art. Such dosage forms include, but are not limited to, injections, sterile powders, external preparations, and the like. In some embodiments, the pharmaceutical composition may be administered via a parenteral route selected from the group consisting of subcutaneous injection, intraepidermal injection, intradermal injection, and intralesional injection.

In some embodiments, the pharmaceutical composition further comprises a pharmaceutically acceptable carrier commonly used in pharmaceutical manufacturing. For example, the pharmaceutically acceptable carrier may comprise one or more of the following agents: solvents, buffers, emulsifiers, suspending agents, decomposers, disintegrating agents, dispersing agents, binding agents, excipients, stabilizing agents, chelating agents, diluents, gelling agents, preservatives, wetting agents, lubricants, absorption delaying agents, liposomes, and the like. The selection and amounts of such agents fall within the professional judgment and routine skill of those skilled in the art.

In some embodiments, the pharmaceutically acceptable carrier comprises a solvent selected from the group consisting of water, normal saline, phosphate-buffered saline (PBS), and aqueous solutions containing alcohol.

In some embodiments, the foregoing composition may be a non-medical edible composition. In some embodiments, the edible composition may be formulated as a food product or a food additive. That is, the edible composition may be added to food materials during food preparation using conventional methods, or incorporated during the manufacturing process of a food product. As used herein, the food product refers to a product formulated with edible materials for consumption by humans or animals.

In some embodiments, the food product includes, but is not limited to, beverages, fermented foods, bakery products, non-medical health foods, and non-medical dietary supplements.

### Example 1: Preparation of Samples

### 1-1. Preparation of ginseng extract:

Whole fleshy roots of ginseng (artificially cultivated for less than five years and frozen within seven days after harvesting) were pulverized into a powder. The powder was passed through a 12-mesh screen to obtain ginseng powder having a particle size of less than 12 mm. The ginseng powder was then mixed with water as an extraction solvent at a weight ratio of 1:5, followed by extraction at 85° C. for 1 hour to obtain a primary extract. The primary extract was subsequently passed through a 400-mesh screen to obtain the ginseng extract.

### 1-2. Preparation of white ginseng extract:

White ginseng was pulverized into a powder and passed through a 12-mesh screen to obtain white ginseng powder having a particle size of less than 12 mm. The white ginseng powder was then mixed with water as an extraction solvent at a weight ratio of 1:5, followed by extraction at 85° C. for 1 hour to obtain a primary extract. The primary extract was subsequently passed through a 400-mesh screen to obtain the white ginseng extract. As used herein, white ginseng refers to ginseng obtained by peeling fresh ginseng and drying the peeled ginseng using hot air at a temperature ranging from 45° C. to 50° C.

### 1-3. Preparation of red ginseng extract:

Red ginseng was pulverized into a powder and passed through a 12-mesh screen to obtain red ginseng powder with a particle size of less than 12 mm. The red ginseng powder was then mixed with water as an extraction solvent at a weight ratio of 1:5 and extracted at 85° C. for 1 hour to obtain a primary extract. The primary extract was subsequently passed through a 400-mesh screen to obtain the red ginseng extract. As used herein, red ginseng refers to fresh ginseng that has not been peeled, steamed at 90-100° C. for at least 1 hour, and then dried using hot air at 45-50° C.

### Example 2: Component analysis of ginseng extract

To evaluate the differences in composition of ginseng processed into white ginseng and red ginseng, the content of ginsenoside Rg3 and fingerprint chromatogram of ginseng, white ginseng, and red ginseng were analyzed.

### 2-1. Instrumentation and conditions:

Nuclear Magnetic Resonance (NMR) Spectrometer: 1D and 2D spectra were recorded using an Ascend 400 MHz instrument (Bruker Co., Germany), with chemical shifts (δ) reported in parts per million (ppm).

Mass Spectrometer (MS): Tandem MS with two-dimensional ion trap Fourier transform MS and ESI-MS/MS was performed using a Bruker amaZon SL system, reporting values in m/z.

Medium Pressure Liquid Chromatography (MPLC): CombiFlash^{®} Rf+ system (Teledyne ISCO, Lincoln, NE, USA).

High Performance Liquid Chromatography (HPLC): Agilent 1200 series; degassing by Agilent vacuum degasser 1322A; solvent delivery by Agilent quaternary pump G1311A; Multiple Wavelength Detector (MWD) Agilent G1314B; Diode Array Detector (DAD) Agilent 1260 Infinity DAD VL G1315D, detection wavelengths 210 nm, 280 nm, 320 nm, and 365 nm (Agilent, Germany).

Analytical Column: Luna^{®} 5 µm C18 (2), 100 Å, 250 × 10 mm (Phenomenex, USA).

Column Chromatography Materials: Sephadex LH-20 (Pharmacia, Piscataway, NJ, USA), Diaion HP-20 (Mitsubishi Chemical Co., Japan), Merck Kieselgel 60 (40-63 µm, Art. 9385), Merck LiChroprep^{®} RP-18 (40-63 µm, Art. 0250).

Thin-Layer Chromatography (TLC) Plates: TLC aluminium sheets (Silica gel 60 F254, 0.25 mm, Merck, Germany) and TLC aluminium sheets (RP-18 F254-S, 0.25 mm, Merck, Germany).

### Solvents and sources:

n-Hexane, ethyl acetate, acetone, methanol, ethanol, acetonitrile (all purchased from Merck Taiwan); chloroform-d1 (deuteration degree 99.5%), methanol-d4 (deuteration degree 99.5%), deuterium oxide (D₂O, deuteration degree > 99.8%), dimethyl sulfoxide-d6 (DMSO-d6, deuteration degree > 99.9%) (Merck Taiwan).

### 2-2. Analysis procedure

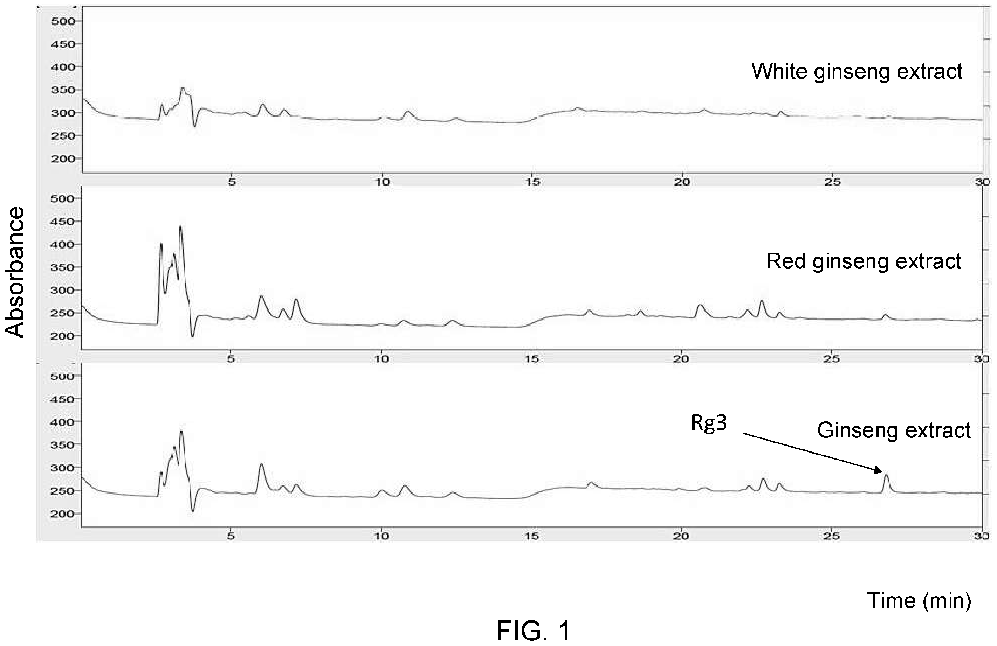

The ginseng extract, white ginseng extract, and red ginseng extract prepared in Example 1 were analyzed by High Performance Liquid Chromatography (HPLC) to obtain individual fingerprint chromatogram, as shown in FIG. 1.

The HPLC conditions were as follows: the mobile phase consisted of methanol (solvent A) and water (solvent B) with 0.1% formic acid added; the flow rate was 1 mL/min; the column temperature was maintained at 40° C.; and the injection volume was 10 µL. Prior to injection, the ginseng extracts were filtered through a 0.22 µm polyvinylidene fluoride (PVDF) membrane.

The elution gradient was set as follows: at 0 minutes, A:B = 2:98; at 10 minutes, A:B = 2:98; at 40 minutes, A:B = 70:30; at 50 minutes, A:B = 100:0; and at 60 minutes, A:B = 100:0.

Quantitative analysis of ginsenoside Rg3 was also performed for the ginseng extract, red ginseng extract, and white ginseng extract. A standard curve was prepared using a ginsenoside Rg3 reference compound (purchased from Merck Taiwan, CAS No. 14197-60-5, purity ≥ 98%).

To prepare the standard solution, 10 mg of ginsenoside Rg3 was accurately weighed and dissolved in a 1:1 (v/v) methanol/water solution, then diluted to 10 mL in a volumetric flask, resulting in a concentration of 100 mg/100 mL. Calibration solutions were prepared at concentrations of 40, 20, 10, 5, and 1 mg/100 mL. HPLC analysis was performed using a Mightysil RP-18 GP 250 column (250 × 4.6 mm, 5 µm, Kanto, Tokyo, Japan), with detection at 210 nm. The resulting peak areas for 40, 20, 10, 5, and 1 mg/100 mL were 2206.5, 1108.1, 477.7, 230.4, and 48.1, respectively. Linear regression analysis of peak area (y-axis) versus concentration (x-axis) yielded the standard curve: y = 43.84x + 16.275 (r² = 0.9998).

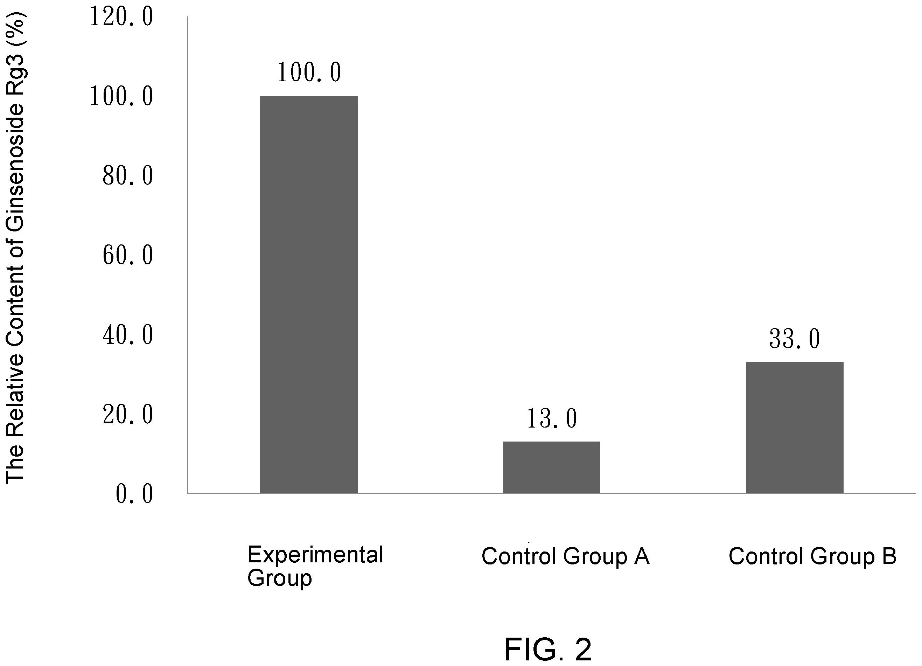

Based on the fingerprint profiles, the peak areas of ginseng extract, red ginseng extract, and white ginseng extract were 599.4, 208.7, and 92.1, respectively. Using the standard curve, the ginsenoside Rg3 content of the ginseng extract, red ginseng extract, and white ginseng extract was calculated to be 13.3 mg/100 mL, 4.389 mg/100 mL, and 1.792 mg/100 mL, respectively. The relative percentages are shown in FIG. 2.

### 2-3. Results

Referring to FIG. 1, the peak appearing at 27 minutes corresponds to the bioactive compound ginsenoside Rg3. As shown in FIG. 1, the ginseng extract exhibits the highest peak at 27 minutes, indicating that the ginseng extract contains the highest content of ginsenoside Rg3. The red ginseng extract shows a smaller peak near 27 minutes, indicating that red ginseng extract also contains ginsenoside Rg3, but at a lower content compared to the ginseng extract. Similarly, the white ginseng extract shows a small peak near 27 minutes, indicating that its ginsenoside Rg3 content is also relatively low.

Referring to FIG. 2, the ginsenoside Rg3 content of the ginseng extract (experimental group) was set as 100%. Relative to the ginseng extract, the ginsenoside Rg3 content of the red ginseng extract (control group B) is 13.0%, and the ginsenoside Rg3 content of the white ginseng extract (control group A) is 3.3%.

### Example 3: Total Alkaloid Content Test

### 3-1. Materials

DR solution: Prepared by mixing Solution 1 (0.8 g of bismuth nitrate pentahydrate in 40 mL of distilled water and 10 mL of glacial acetic acid) with Solution 2 (8.0 g of potassium iodide in 20 mL of distilled water).

Standard bismuth nitrate [Bi(NO₃)₃·5H₂O] solution: Prepared by dissolving 10 mg of bismuth nitrate in 5 mL of concentrated nitric acid, then diluting with distilled water to 100 mL.

3% thiourea solution: Prepared by dissolving 3 g of thiourea in 100 mL of distilled water.

1% disodium sulfide solution: Prepared by dissolving 1 g of disodium sulfide in 100 mL of distilled water

### 3-2. Preparation of standard curve

To prepare the standard curve, 1 mL, 2 mL, 3 mL, 4 mL, 5 mL, 6 mL, 7 mL, 8 mL, and 9 mL of the standard bismuth nitrate solution were transferred into individual 10 mL volumetric flasks and diluted with distilled water to the 10 mL mark to generate nine standard solutions of varying bismuth nitrate concentrations. Next, 1 mL of each of the nine standard solutions was mixed with 5 mL of the 3% thiourea solution to generate nine yellow standard samples. The absorbance of each yellow standard sample was measured at 435 nm relative to a colorless reagent blank.

### 3-3. Experimental Procedure

For each group, 5 mL of sample was taken, and HCl was added to maintain the pH at 2.0-2.5. The experimental group sample was the ginseng extract prepared according to Example 1, control group A sample was the white ginseng extract prepared according to Example 1, and control group B sample was the red ginseng extract prepared according to Example 1.

To each sample, 2 mL of DR solution was added, followed by centrifugation to form a precipitate in the centrifuged solution. To ensure complete precipitation, additional DR solution was added to the centrifuged solution.

After centrifugation, the supernatant was decanted, and the precipitate was collected and further washed with alcohol.

After removing the filtrate, the precipitate was treated with 2 mL of 1% disodium sulfide solution, forming a brown-black precipitate. To confirm completion of precipitation, an additional 2 drops of disodium sulfide solution were added.

The remaining solid was dissolved in 2 mL of concentrated nitric acid, with heating applied if necessary to achieve complete dissolution. The solution was then diluted with distilled water to 10 mL in a volumetric flask. Subsequently, 1 mL of this solution was mixed with 5 mL of thiourea solution, and the absorbance was measured at 435 nm.

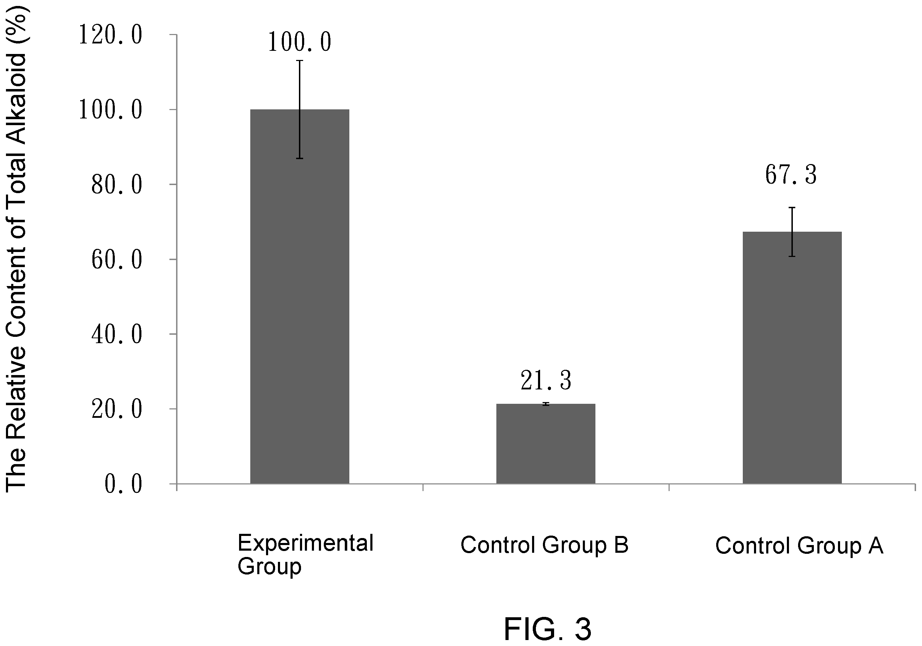

The total alkaloid content of the samples was determined by comparing the absorbance values with the standard curve. The results are shown in FIG. 3.

### 3-4. Results

Referring to FIG. 3, the absorbance of the experimental group was set as 100%. Compared to the experimental group, the total alkaloid content of control group A is 67.3%, and the total alkaloid content of control group B is 21.3%.

### Example 4: Antioxidant Efficiency Test

### 4-1. Materials and Equipment

Cell line: Skeletal muscle cells C2C12 (hereinafter referred to as C2C12 cells) were obtained from the American Type Culture Collection (ATCC^{®}), C2C12 cell line (ATCC CRL-1772^{™}).

Cell culture medium: Minimum essential medium (MEM) supplemented with 10% fetal bovine serum (FBS, Gibco, Cat. No. 10437-028), 1% Antibiotic-Antimycotic (Gibco, Cat. 15140122), and 1 mM sodium pyruvate (Gibco, Cat. 11360-070) (hereinafter referred to as MEM medium).

Phosphate-buffered saline (PBS): Purchased from Gibco, Cat. No. 10437-028.

DCFH-DA solution: 2,7-dichloro-dihydro-fluorescein diacetate (DCFH-DA, Sigma, Cat. SI-D6883) was dissolved in dimethyl sulfoxide (DMSO, Sigma, Cat. SI-D6883-50MG) to prepare a 5 µg/mL solution.

Flow cytometer: BD Accuri C6 Plus.

Hydrogen peroxide (H₂O₂): Sigma-Aldrich, Cat. No. 95299-1L.

Trypsin-EDTA: 10X Trypsin-EDTA (Gibco) diluted 10-fold with 1X PBS solution.

### 4-2. Experimental Procedure

C2C12 cells were seeded into a 6-well plate at a density of 2 × 10⁵ cells/well, with 2 mL of MEM medium added per well, and incubated at 37°C in a CO₂ incubator for 24 hours.

After cell attachment, C2C12 cells were divided into an experimental group, control group A, and control group B. The culture medium of each group was removed and replaced with 500 µL/well of test medium, followed by incubation at 37°C for 1 hour.

In some embodiments, the culture medium for the experimental group comprises 0.25% ginseng extract prepared according to Example 1 and 1 mM hydrogen peroxide in cell culture medium. The culture medium for control group A comprises 0.25% white ginseng extract prepared according to Example 1 and 1 mM hydrogen peroxide in cell culture medium. The culture medium for control group B comprises 0.25% red ginseng extract prepared according to Example 1 and 1 mM hydrogen peroxide in cell culture medium.

In some embodiments, DCFH-DA solution was added, and the cells were incubated at 37°C for 15 minutes.

In some embodiments, 10 µL of hydrogen peroxide was added to each well, followed by incubation at 37°C for 1 hour.

The culture medium was removed, and the cells were washed twice with PBS.

The medium was removed, and 200 µL/well of 1X trypsin-EDTA was added and incubated in the dark for 5 minutes.

The reaction was terminated with culture medium, and the cells were transferred to 1.5 mL microcentrifuge tubes and centrifuged at 400 × g for 10 minutes.

The supernatant was removed, and the cells were washed with PBS and centrifuged again at 400 × g for 10 minutes.

The supernatant was removed, and the cells were resuspended in 1 mL of PBS per tube.

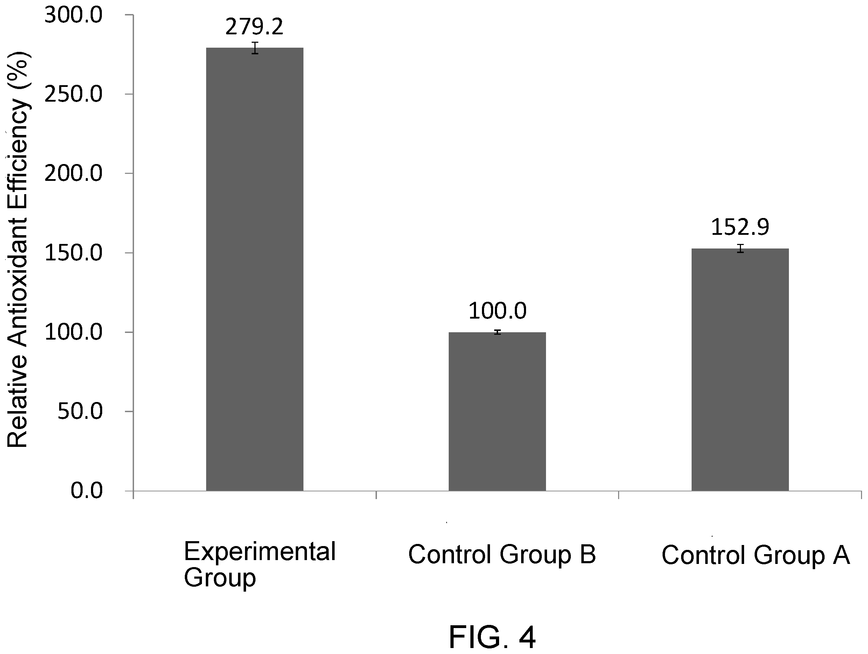

The fluorescence signal of DCFH-DA was measured using a flow cytometer (excitation: 450-490 nm; emission: 510-550 nm). The results are shown in FIG. 4, where antioxidant efficiency is presented as relative values.

Referring to FIG. 4, the value of control group B was set as 100%. Compared to control group B, the relative antioxidant efficiency of the experimental group reached 279.2%, while that of control group A was 152.9%. These results indicate that the ginseng extract significantly enhances the resistance of skeletal muscle cells to oxidative damage induced by hydrogen peroxide, and the effect is approximately 2.7 times higher than that of red ginseng.

### Example 5: Mitochondrial Activity Assay

Mitochondria are essential organelles responsible for cellular metabolism and energy production, and reduced mitochondrial activity generally indicates a fatigued cellular state. In this assay, flow cytometry was employed to evaluate changes in mitochondrial activity of C2C12 skeletal muscle cells after treatment with different samples. JC-1 aggregation behavior was used as an indicator of mitochondrial activity. In healthy mitochondria with normal membrane potential, JC-1 exists in a polymeric form, whereas a decrease in mitochondrial membrane potential results in the conversion of JC-1 into a monomeric form. Accordingly, the aggregation state of JC-1 serves as an indicator for evaluating mitochondrial activity.

### 5-1. Materials and Instruments

Experimental cell line: Skeletal muscle C2C12 cells (hereinafter referred to as "C2C12 cells"), purchased from the American Type Culture Collection (ATCC^{®}), C2C12 cell line (ATCC CRL-1772^{™}).

Culture medium: Dulbecco's Modified Eagle's Medium (DMEM), purchased from Gibco, USA (Cat. No. 11965-092), supplemented with 10% fetal bovine serum (FBS) (Gibco, USA; Cat. No. 10437-028) and 1% antibiotics (Gibco, USA; Cat. No. 15240-062).

Mitochondrial membrane potential detection kit: BDTM MitoScreen (JC-1) Kit (BD Biosciences; Cat. No. 551302), comprising lyophilized JC-1 dye and 10× assay buffer. Prior to use, the 10× assay buffer was diluted tenfold with 1× PBS to obtain a 1× assay buffer. The JC-1 dye was reconstituted by adding 130 µL of DMSO to form a JC-1 stock solution, which was subsequently diluted with the 1× assay buffer at a ratio of 1:100 to prepare a JC-1 working solution.

Trypsin: 10× Trypsin-EDTA (Gibco), diluted tenfold with 1× PBS.

Flow cytometer: Flow cytometry system (Beckman).

### 5-2. Experimental Procedure

C2C12 cells were seeded into six-well culture plates at a density of 1 × 10⁵ cells per well and cultured at 37°C for 24 hours. The cultured cells were divided into three groups: an experimental group, control group A, and control group B. The culture medium was removed and replaced with test media, followed by incubation at 37°C for 24 hours.

The test medium for the experimental group comprised culture medium containing 0.25% ginseng extract prepared according to Example 1. The test medium for control group A comprised culture medium containing 0.25% white ginseng extract prepared according to Example 1. The test medium for control group B comprised culture medium containing 0.25% red ginseng extract prepared according to Example 1.

After incubation, the test media were removed, and the cells were washed twice with 1× PBS.

Two hundred microliters of trypsin were added to each well and allowed to react in the dark for 5 minutes. The reaction was terminated by adding culture medium. The cells and medium from each well were collected into corresponding 1.5 mL centrifuge tubes and centrifuged at 400 × g for 10 minutes.

After centrifugation, the supernatant was removed, and the cell pellets were resuspended in 1 mL of 1× PBS or transferred into 15 mL centrifuge tubes to form cell suspensions.

The cell suspensions were centrifuged at 400 × g for 5 minutes.

After centrifugation, the supernatant was removed, and 100 µL of JC-1 working solution was added to each centrifuge tube.

The cell pellets were vortexed with the JC-1 working solution and incubated in the dark for 15 minutes.

After incubation, the samples were centrifuged at 400 × g for 5 minutes.

The supernatant was removed, the cell pellets were resuspended in 1 mL of 1× PBS, and the samples were centrifuged again at 400 × g for 5 minutes.

The washing step with 1× PBS was repeated, followed by centrifugation at 400 × g for 5 minutes.

After removal of the supernatant, the cells were resuspended in 500 µL of 1× PBS to obtain cell suspensions for analysis.

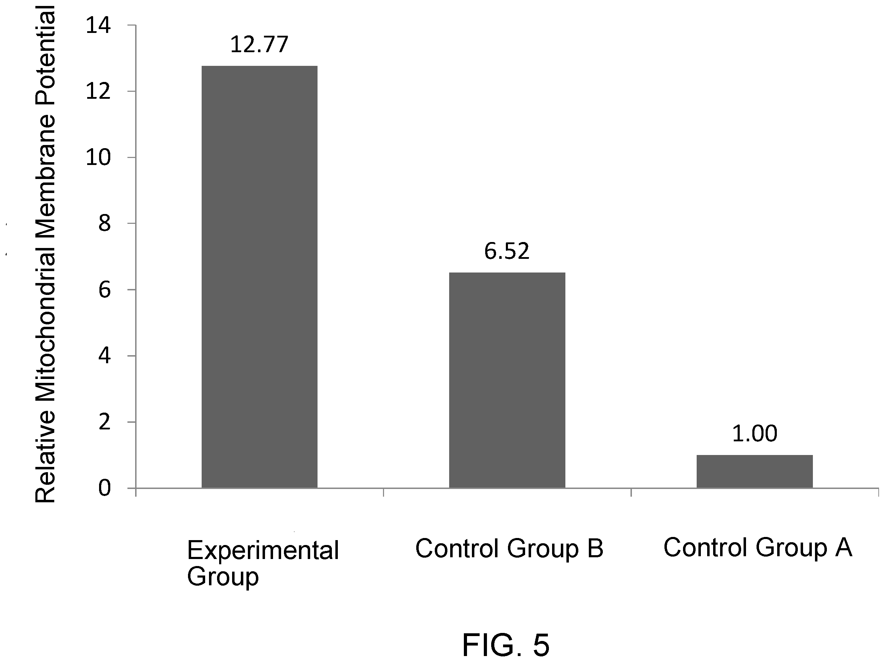

Mitochondrial membrane potential of the cells was measured by flow cytometry to evaluate mitochondrial activity. Each experiment was performed in triplicate, and the results were averaged. The average value of control group A was set as 1 to calculate relative JC-1 aggregation levels, and the relative JC-1 aggregation levels of the experimental group and control group B were calculated accordingly, as shown in FIG. 5.

### 5-3. Results

Referring to FIG. 5, the value of control group A was set as 1. Compared to control group A, the experimental group exhibited a relative mitochondrial membrane potential of 12.77, while control group B exhibited a relative mitochondrial membrane potential of 6.52. These results indicate that the ginseng extract significantly enhances mitochondrial activity in skeletal muscle cells and mitigates the reduction in mitochondrial function associated with excessive fatigue.

### Example 6: Skeletal Muscle Cell Proliferation Assay

Individuals with a greater amount of muscle tissue generally exhibit higher endurance and are capable of sustaining physical activity for a longer period without experiencing fatigue.

### 6-1. Materials and Equipment

Experimental cell line: Skeletal muscle cells C2C12 (hereinafter referred to as "C2C12 cells") were used. The C2C12 cell line was obtained from the American Type Culture Collection (ATCC^{®}), catalog number ATCC CRL-1772^{™}.

Culture medium: Dulbecco's Modified Eagle's Medium (DMEM) was used, purchased from Gibco (USA), catalog no. 11965-092, supplemented with 10% fetal bovine serum (FBS) (Gibco, USA; catalog no. 10437-028) and 1% antibiotics (Gibco, USA; catalog no. 15240-062).

Cell proliferation assay kit: ELISA-based BrdU cell proliferation kit (Roche; catalog no. 11647229001), comprising an EdU reagent, a FixDenat solution, a BrdU-POD reagent, washing solution, and a substrate solution.(After removal of the antibody conjugate, wells were rinsed three times with 200-300 µL of washing solution.)

### 6-2. Experimental Procedure

First, 5,000 cells were seeded into each well of a 96-well culture plate and incubated at 37 °C for 2 hours. The cultured cells were then divided into five groups: an experimental group, a blank group, a positive control group, control group A, and control group B.

The blank group received only culture medium and was incubated at 37 °C for 24 hours. The positive control group additionally received 10% FBS. The experimental group received culture medium containing 0.25% of the ginseng extract obtained in Example 1. Control group A received culture medium containing 0.25% of the white ginseng extract obtained in Example 1. Control group B received culture medium containing 0.25% of the red ginseng extract obtained in Example 1.

Subsequently, the EdU reagent was added in accordance with the standard protocol of the cell proliferation assay kit, followed by incubation at 37 °C for 24 hours. The supernatant was then removed, and the FixDenat solution was added and allowed to react at room temperature for 30 minutes. After removal of the FixDenat solution, the cells were washed once with PBS buffer. The BrdU-POD reagent was added and incubated at room temperature for 90 minutes. The antibody conjugate (BrdU-POD reagent) was removed, and the wells were rinsed three times with washing solution. After removal of the washing solution, the substrate solution was added and incubated at room temperature for 15 minutes. Subsequently, 1 M H₂SO₄ was added and allowed to react for 10 minutes. After shaking at 300 rpm for 1 minute, the fluorescence signal of the cells in each well was measured (excitation wavelength: 450 nm), and the number of cells was calculated accordingly.

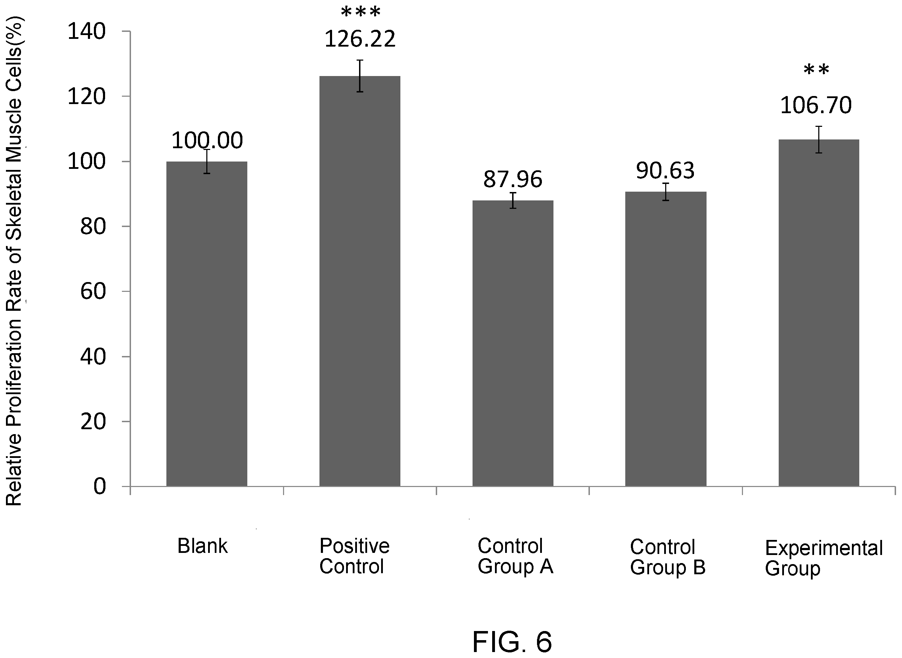

The experimental results were analyzed using Student's *T*-test with Excel software to determine whether statistically significant differences existed between sample groups, as shown in FIG. 6. In FIG. 6, "*" indicates a p-value less than 0.05, "**" indicates a p-value less than 0.01, and "***" indicates a p-value less than 0.001. A greater number of asterisks represents a higher level of statistical significance relative to the blank group.

### 6-3. Results

Referring to FIG. 6, when the number of skeletal muscle cells measured in the blank group is defined as 100%, the relative number of skeletal muscle cells in the positive control group was 126.22%, while the relative number of skeletal muscle cells in the experimental group was 106.7%. Compared with the blank group, the experimental group exhibited a statistically significant difference.

However, the white ginseng extract (control group A) and the red ginseng extract (control group B) not only failed to promote skeletal muscle cell proliferation, but instead resulted in a reduction in the number of skeletal muscle cells. Accordingly, it is demonstrated that only the ginseng extract is capable of effectively promoting skeletal muscle cell growth, thereby enhancing anti-fatigue efficiency in an individual.

### Example 7: Human Study

Sample: ginseng extract prepared according to the procedure in Example 1 was used.

Three human subjects were enrolled. Each subject was aged between 20 and 55 years. The subjects self-reported as being prone to fatigue and frequent brain fog and were healthy adults without other underlying diseases.

### 7-1. Test Items

The test items included: blood oxygen saturation (SpO₂, oxygen saturation expressed as a percentage), a lipid peroxidation marker (MDA, malondialdehyde), total antioxidant capacity (TAC), a judgment test (Stroop Test), and a short-term memory test (Digit Span Test).

Blood oxygen saturation refers to the ratio of oxygenated hemoglobin to total hemoglobin in blood and was measured using a pulse oximeter (Wistron handheld type, model TD-8255). During the test, each subject continuously wore a mask to control oxygen supply and was exposed to a hypoxic environment. Blood oxygen saturation was recorded before exposure (ambient air, approximately 21% oxygen) and after 30 minutes of hypoxic exposure (7% oxygen).

The lipid peroxidation marker was assessed using malondialdehyde (MDA), one of the terminal products of lipid peroxidation, as an indicator. Blood samples were collected and analyzed by an accredited clinical testing laboratory (Li-Ren Medical Laboratory).

Total antioxidant capacity was evaluated using the TPTZ redox method. Based on the reducing capability of antioxidants present in human serum, ferric iron complexes (Fe³⁺-TPTZ) are reduced to ferrous iron complexes (Fe²⁺-TPTZ), thereby enabling assessment of total antioxidant capacity in blood.

The judgment test (Stroop Test) was evaluated based on the time required to complete the test, wherein a shorter response time indicates better judgment ability. In this test, a color word was displayed, and the font color of the word did not necessarily correspond to the meaning of the word itself. The subject was required to identify the font color rather than the word meaning. A total of 40 questions were administered consecutively, and the total time required to complete the test was recorded. A shorter completion time represents better judgment ability.

Short-term memory was evaluated based on the number of correct answers and answer accuracy, wherein a higher number of correct answers and higher accuracy indicate better short-term memory. The Digit Span Test was employed for this evaluation. Subjects were required to memorize a sequence of numbers and then recall the sequence in reverse order. The length of the number sequence was gradually increased until the subject could no longer correctly recall the sequence. Answer accuracy was calculated by dividing the number of correct answers by the total number of test items and multiplying by 100 to obtain an average accuracy percentage.

### 7-2. Experimental Procedure

Each of the three subjects ingested 5 mL of the ginseng extract daily for four consecutive weeks. Measurements were conducted before ingestion (Week 0, serving as the control group) and after four weeks of ingestion (Week 4, serving as the experimental group) using the above-described equipment and methods.

### 7-3. Results

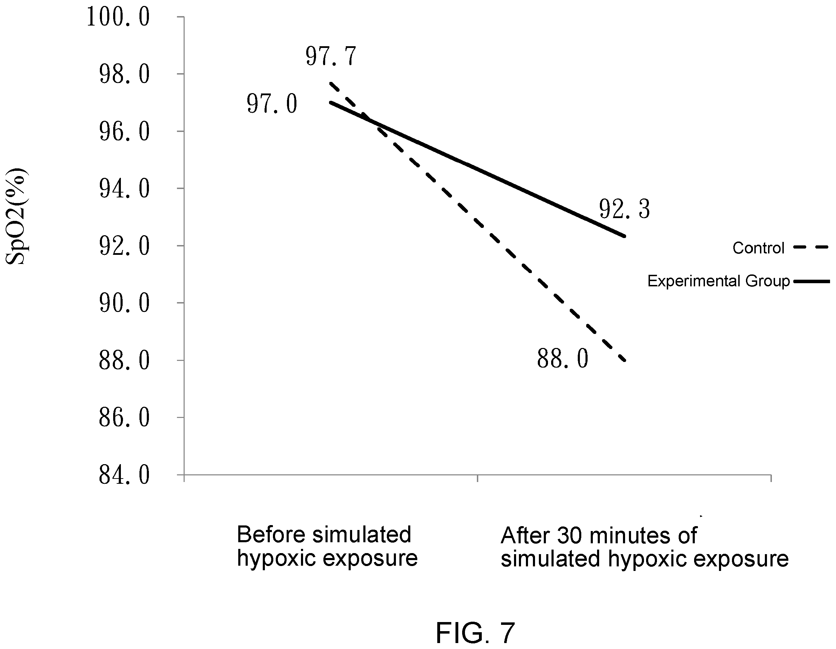

Referring to FIG. 7, the average blood oxygen saturation of the control group decreased from 97.7% to 88.0% after 30 minutes of simulated hypoxic exposure, representing a difference of 9.7 percentage points. After four weeks of daily ingestion of 5 mL of the ginseng extract, the average blood oxygen saturation of the three subjects decreased from 97.0% to 92.3% after 30 minutes of hypoxic exposure, representing a difference of 4.7 percentage points. A smaller difference indicates better hypoxia tolerance. Accordingly, after only four weeks of administration of the ginseng extract of the present invention, the subjects were able to maintain blood oxygen saturation above 90% even after 30 minutes under hypoxic conditions, demonstrating a significant improvement in hypoxia tolerance. That is, daily ingestion of 5 mL of the ginseng extract effectively enhances hypoxia tolerance.

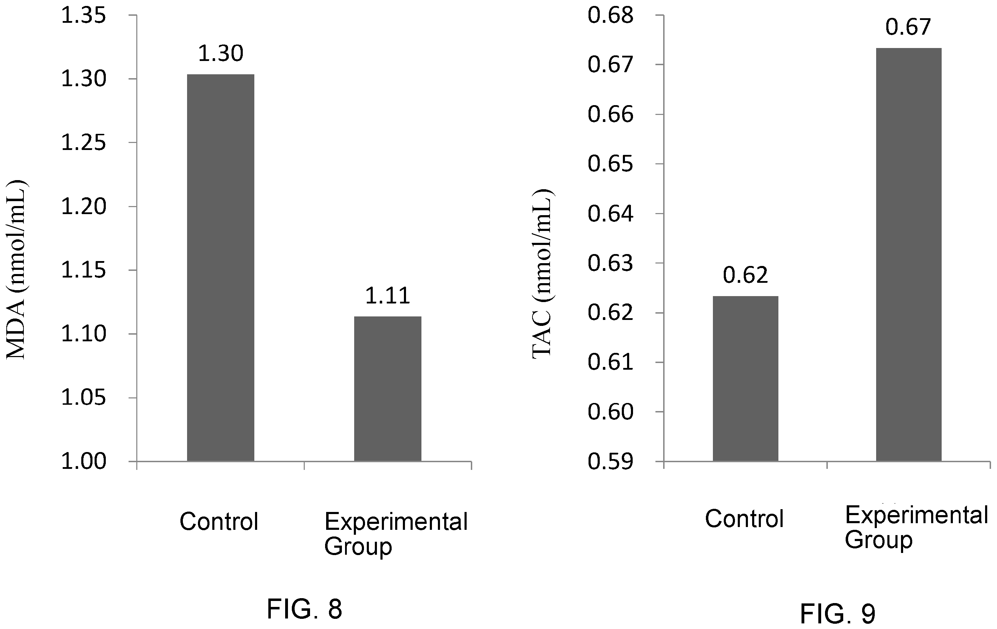

Referring to FIG. 8, after four weeks of daily ingestion of 5 mL of the ginseng extract, the average lipid peroxidation marker value of the three subjects decreased from 1.3 at Week 0 to 1.11 at Week 4 while maintaining normal daily routines. That is, daily ingestion of 5 mL of the ginseng extract effectively improved the lipid peroxidation marker by 14.58%.

Referring to FIG. 9, after four weeks of daily ingestion of 5 mL of the ginseng extract, the average total antioxidant capacity of the three subjects increased from 0.62 at Week 0 to 0.67 at Week 4 while maintaining normal daily routines. That is, daily ingestion of 5 mL of the ginseng extract effectively improved total antioxidant capacity by 8.02%.

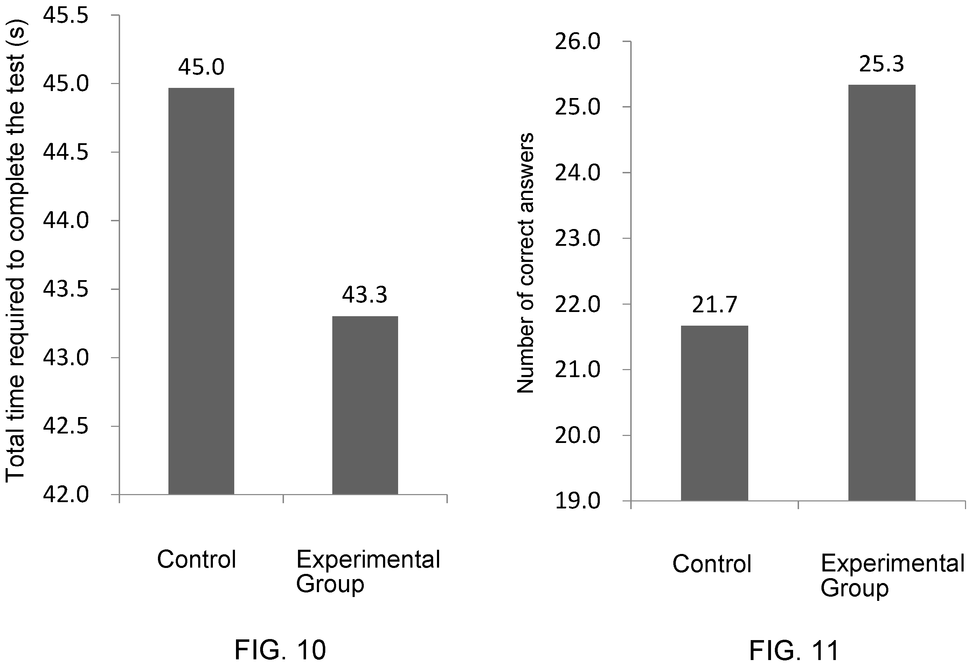

Referring to FIG. 10, after four weeks of daily ingestion of 5 mL of the ginseng extract, the average time required to complete the judgment test decreased from 45 seconds at Week 0 to 43.3 seconds at Week 4 while maintaining normal daily routines. That is, daily ingestion of 5 mL of the ginseng extract effectively improved judgment ability.

Referring to FIG. 11, after four weeks of daily ingestion of 5 mL of the ginseng extract, the average number of correctly answered questions increased from 21.7 at Week 0 to 25.3 at Week 4 while maintaining normal daily routines. That is, daily ingestion of 5 mL of the ginseng extract effectively improved short-term memory.

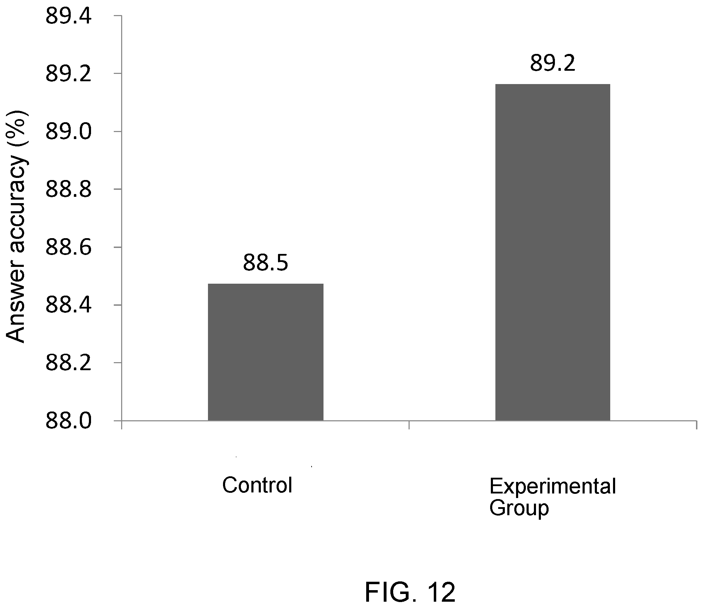

Referring to FIG. 12, after four weeks of daily ingestion of 5 mL of the ginseng extract, the average answer accuracy increased from 88.5% at Week 0 to 89.2% at Week 4 while maintaining normal daily routines. That is, daily ingestion of 5 mL of the ginseng extract effectively improved short-term memory.

In summary, the ginseng extract of any embodiment may be used to prepare a composition for anti-fatigue purposes or for enhancing cognitive performance. The ginseng extract of any embodiment may be used to prepare a composition for promoting mitochondrial activity, promoting skeletal muscle cell proliferation, improving hypoxia tolerance, improving blood oxygen saturation, improving lipid peroxidation marker MDA levels, improving total antioxidant capacity (TAC), improving judgment ability, and enhancing short-term memory.

## Claims

1. A use of ginseng extract for preparing an anti-fatigue composition, wherein the ginseng extract is extracted from *Panax ginseng C. A*. *Meyer* using water as an extraction solvent.

2. The use according to claim 1, wherein the ginseng extract enhances mitochondrial activity.

3. The use according to claim 1, wherein the ginseng extract promotes proliferation of skeletal muscle cells.

4. A use of ginseng extract for preparing a composition for improving cognitive function, wherein the ginseng extract is extracted from *Panax ginseng C. A. Meyer* using water as extraction solvent.

5. The use according to claim 4, wherein the ginseng extract improves hypoxia tolerance.

6. The use according to claim 4, wherein the ginseng extract improves blood oxygen saturation level.

7. The use according to claim 4, wherein the ginseng extract improves lipid peroxidation by increasing a level of malondialdehyde (MDA).

8. The use according to claim 4, wherein the ginseng extract improves total antioxidant capacity (TAC).

9. The use according to any of claims 1 to 8, wherein the composition is a food composition, and the composition comprises at least 5 mL of the ginseng extract.

10. The use according to any of claims 1 or 4, wherein the ginseng extract comprises at least 13.3 mg of ginsenoside Rg3.
